# EUROPEAN PATENT SPECIFICATION

(11) **EP 4 037 633 B1**
(45) Date of publication and mention of the grant of the patent: **13.05.2026**
(21) Application number: 20872525.9
(22) Date of filing: 25.09.2020
(51) Int. Cl.: A61J 1/20, A61M 5/31, A61M 5/00, A61M 5/315

(54) **MEDICAMENT FILLING SYSTEM**
MEDIKAMENTENABFÜLLSYSTEM
SYSTÈME DE REMPLISSAGE DE MÉDICAMENT

(30) Priority: 30.09.2019 US 201962908343 P
(43) Date of publication of application: 10.08.2022
(60) Divisional application: 26170312.8
(73) Proprietor: Becton, Dickinson and Company, Franklin Lakes, NJ 07417-1880 (US)
(72) Inventor: GYORY, J. Richard, Sudbury, Massachusetts 01776 (US); NEWBY, Mark, Kamas, Utah 84036 (US); WILKINSON, Bradley, Haledon, New Jersey 07508 (US); PETERSON, Bart, Farmington, Utah 84025 (US); PIZZOCHERO, Alessandro, Chelmsford, Massachusetts 01824 (US); GRIGORYANTS, Sergey, Malden, Massachusetts 02148 (US); YU, Bo Yang, Winchester, Massachusetts 01890 (US); DANI, Heena, Middlesex, New Jersey 08846 (US)
(74) Representative: dompatent
(86) International application number: PCT/US2020/052747
(87) International publication number: WO 2021/067133

(56) References cited:
- EP-B1- 0 327 519
- US-A- 5 637 092
- US-A1- 2010 218 846
- US-A1- 2011 264 069
- US-A1- 2014 276 386
- US-A1- 2015 053 305
- US-A1- 2016 144 105
- US-A1- 2016 144 105
- US-A1- 2018 021 521
- US-A1- 2018 126 085
- US-A1- 2019 216 683
- US-B1- 8 777 906

## Description

### FIELD OF THE INVENTION

Various exemplary embodiments of the invention relate to filling a delivery device with medicament via a medicament filling system such as a syringe assembly.

### BACKGROUND OF THE INVENTION

Systems such as delivery devices and syringe assemblies are typically used to inject medication, such as insulin, into a patient. However, inefficiencies and inconveniences can arise. These challenges include minimizing air entering into the delivery device, preventing medicament backflow from exiting the delivery device during and after the filling step, and improving safety, handling and stability.

US Patent No. US2016/144105A1 discloses a drug delivery system, injection device, transfer apparatus, vial holder and method of administering and transferring a drug are disclosed. The system may include a transfer apparatus and an injection device. The transfer apparatus may have receiving stations for a drug source, such as a vial or vial holder, and for an injection device, and fluid flow pathways for transferring drugs from the source into the injection device. The injection device may include an expandable elastic bladder and an injection cannula that is movable between a plurality of positions.

Patent No. US2015/053305A1 discloses a syringe fill system, which is useful for filling syringes for dental anesthetic applications. The system incorporates capability for "push-pull", "pull-push", "push-push", and "pull-pull" modes of operation, for loading syringes with compositions comprising multiple fluid components. A syringe fillable by such syringe fill systems is described, providing haptic and audible feedback to a user, to aid in administering precise amounts of therapeutic compositions. Also disclosed are cassette assemblies for use in such syringe fill systems. Such cassette assemblies may be formed of plastic and elastomeric materials of construction, as disposable or single-use components of the syringe fill system.

### SUMMARY OF THE INVENTION

It is an aspect of the present invention to provide a medicament filling system and a method of using a medicament filling system in accordance with the independent claims. Preferred aspects are further defined by the dependent claims.

Additional and/or other aspects and advantages of the present invention will be set forth in the description that follows, or will be apparent from the description, or may be learned by practice of the invention.

### BRIEF DESCRIPTION OF THE DRAWINGS

The above aspects and features of the present invention will be more apparent from the description for the exemplary embodiments of the present invention taken with reference to the accompanying drawings, in which:
Figure 1 is a first exemplary embodiment of a schematic of a medicament filling system;
Figure 2 is a perspective view of the medicament filling system of Figure 1;
Figure 3 is a transparent perspective view of the medicament filling system of Figure 2;
Figure 4 is a cross-sectional view of the medicament filling system along line A-A of Figure 2 shown when removing air from a delivery device;
Figure 5 is a cross-sectional view of the medicament filling system along line A-A of Figure 2 shown when delivering medicament to the delivery system;
Figure 6 is a second exemplary embodiment of a schematic of a medicament filling system;
Figure 7 is an exploded view of the medicament filling system of Figure 6;
Figure 8 is an exploded view of a syringe assembly used with the medicament filling system of Figure 7;
Figure 9 is a perspective view of a plunger lock assembly used with the syringe assembly of Figure 8;
Figure 10 is a cross-sectional view of the plunger lock assembly along line B-B of Figure 9;
Figure 11 is a perspective view of a third exemplary embodiment of a syringe assembly attached to a vial via an adapter;
Figure 12 illustrates a first embodiment of an adapter used in the syringe assembly of Figure 11;
Figure 13 illustrates a second embodiment of an adapter used in the syringe assembly of Figure 11;
Figure 14 illustrates a third embodiment of an adapter used in the syringe assembly of Figure 11;
Figure 15 illustrates the adapter used in the syringe assembly of Figure 11 when disengaged from the vial;
Figure 16 is a side perspective view of a fourth exemplary embodiment of a syringe assembly having an integrated adapter attached to a vial;
Figure 17 is a top perspective view of the syringe assembly of Figure 16;
Figure 18 is a bottom perspective view of the syringe assembly of Figure 16 mounted to a delivery device;
Figure 19 is a perspective view of a fifth exemplary embodiment of a syringe assembly without an adapter;
Figure 20 is a perspective view of the syringe assembly of Figure 19 engaged to a vial to fill with medicament;
Figure 21 is a perspective view of the syringe assembly of Figure 19 engaged to a delivery device to transfer medicament;
Figure 22 is an exploded view of a sixth exemplary embodiment of a needleless syringe assembly with an adapter having a needle and a vial;
Figure 23 is a perspective view of the syringe assembly of Figure 22 shown connected to the vial;
Figure 24 is a perspective view of the syringe assembly of Figure 22 shown engaging a delivery device;
Figure 25 is a cross-sectional view of the syringe assembly taken along line C-C of Figure 24 shown engaged to the delivery device;
Figure 26 is a cross-sectional view of a seventh exemplary embodiment of a syringe assembly with an alignment adapter engaged to a delivery device;
Figure 27 is an exploded view of the syringe assembly of Figure 26 and a vial;
Figure 28 illustrates a delivery device configured to engage the syringe assembly of Figure 26; and
Figure 29 is a flow diagram of a method for transferring medicament into a syringe assembly using an adapter and prior to expelling the medicament into a delivery device.

### DETAILED DESCRIPTION OF EXEMPLARY EMBODIMENTS

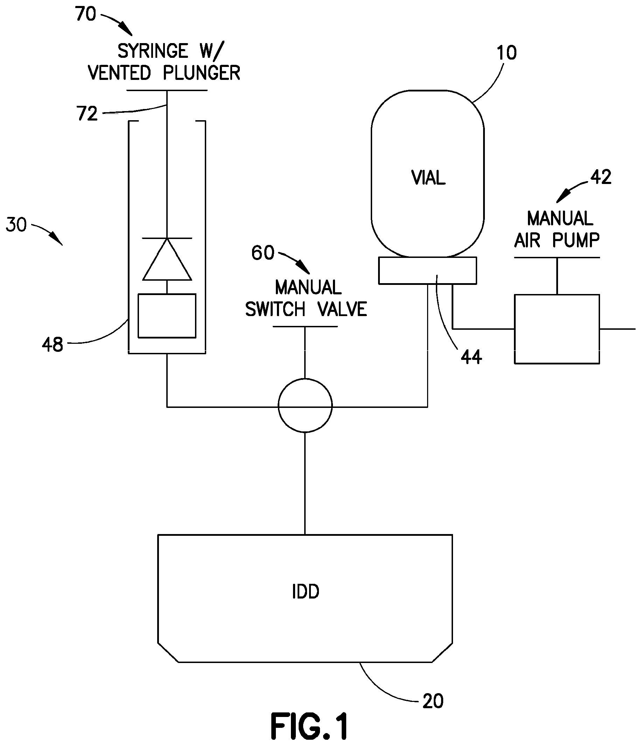

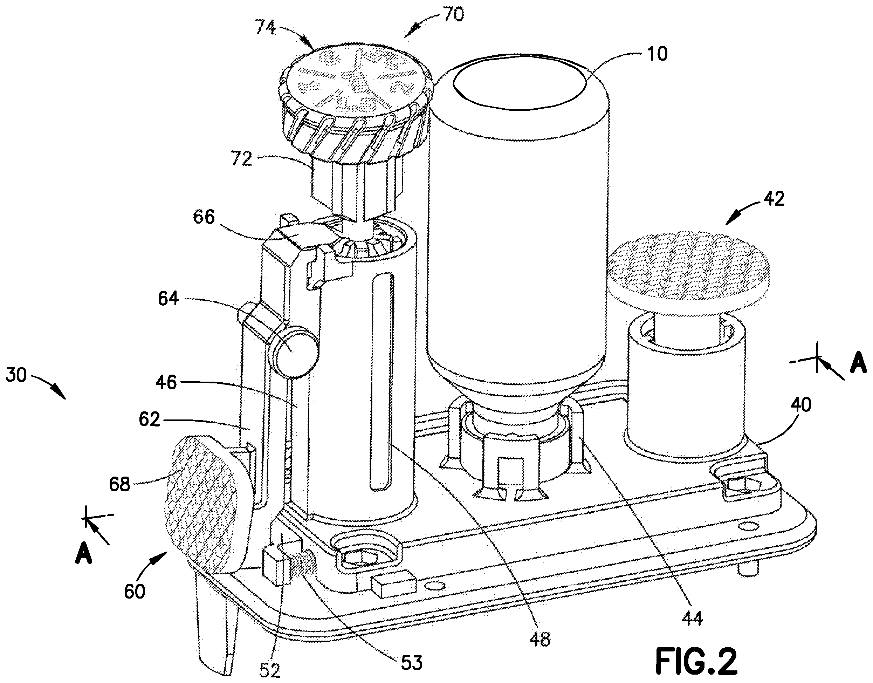

Figures 1-5 illustrate a medicament filling system 30, according to one embodiment. The medicament filling system 30 is configured to engage a vial 10 to receive medicament and an insulin delivery device (IDD) 20 to transfer and fill with the medicament. Figure 1 illustrates a schematic drawing of the overall operation of the medicament filling system 30. Figures 2-5 illustrate a cavity on a bottom surface of the medicament filling system 30 that advantageously provides alignment and is configured to engage the insulin delivery device 20.

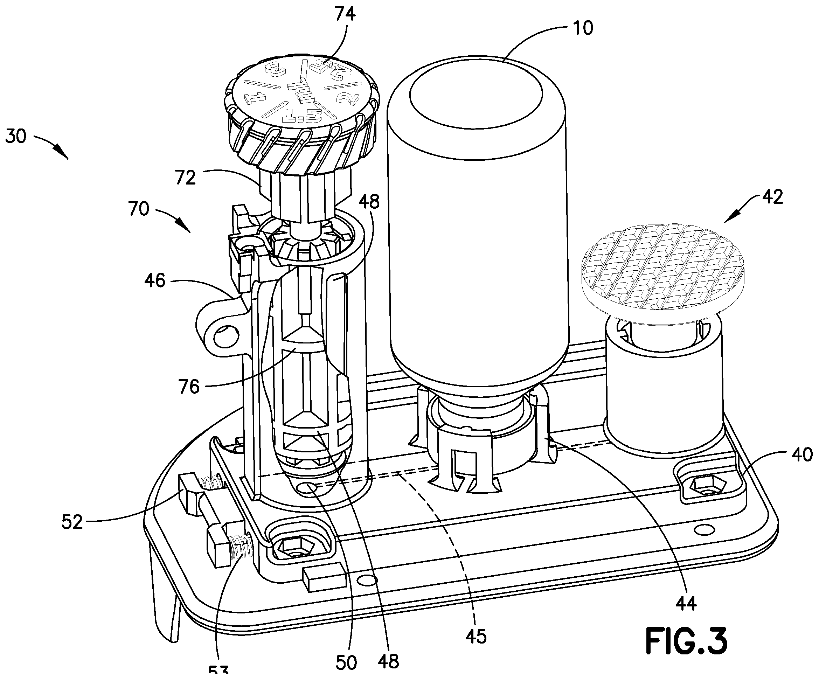

Figures 2 and 3 illustrate that the medicament filling system 30 includes a main housing 40 that includes an air pump 42, a vial adapter 44, a flow path 45, a barrel 46 having a viewing window 48, an outlet port 50 communicating with a needle 54 and a spring element 52 cooperating with a spring 53. The air pump 42 is preferably manually operated but can also be automatically operated. The air pump 42 is in fluid communication with the vial adapter 44.

When a user desires to transfer the medicament from the vial 10 to the barrel 46 in the main housing 10, the vial 10 is attached to the vial adapter 44. The vial adapter 44 includes a dual lumen vial spike that pierces a septum in the vial 10. Subsequently, the user pumps the air pump 42 to supply air to the vial 10. This increased pressure in the vial 10 causes the medicament to exit the vial 10 and enter into the barrel 46 via the flow path 45.

The barrel 46 carries the medicament and includes the viewing window 48 to provide a visual indication of how much medicament is in the barrel 46. Specifically, the viewing window 48 is clear and can include dose markers that are visible when the user looks through the viewing window 48 to see how much medicament is in the barrel 46. The barrel 46 can also include a buoyant colored member that floats on top of the medicament to indicate how much medicament is in the barrel 46 when seen through the viewing window 48. Further details of the syringe barrel components 70 are described below.

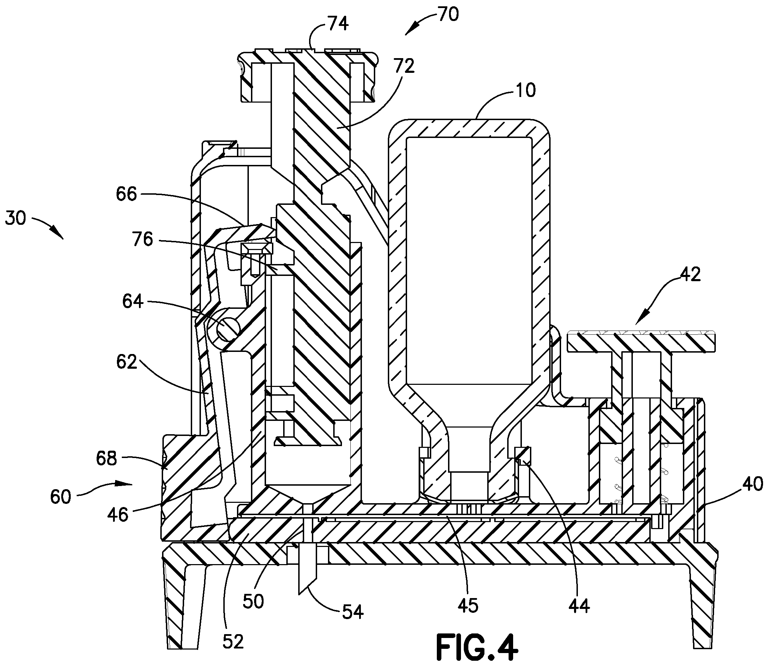

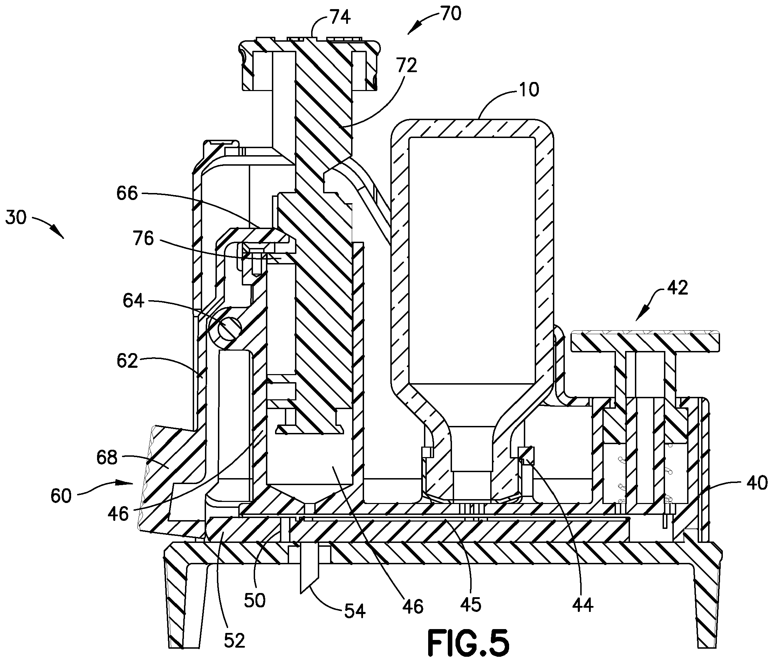

As illustrated in Figures 4 and 5, the main housing 40 also includes an outlet port 50 disposed in the spring element 52 that selectively communicates with the needle 54 and the insulin delivery device 20 based on the movement of a manual switch valve 60. The needle 54 is disposed in the cavity on the bottom surface of the medicament filling system 30. The needle 54 is configured to pierce a septum in the insulin delivery device 20. The manual switch valve 60 advantageously controls a three-way valve and cooperates with a plunger lock feature. Further details of the manual switch valve 60 are described below.

Figure 1 illustrates that the manual switch valve 60 is disposed between the vial adapter 44, the insulin delivery device 20 and the barrel 46 to provide selective fluid communication. Figure 4 illustrates the manual switch valve 60 depressed to move the spring element 52. In this configuration, the main housing 40 is in communication with the insulin delivery device 20 to remove air through the outlet port 50 and the needle 54. Air removal occurs when the user pulls a vented plunger 72 upward. The flow path 45 between the vial adapter 44 and the barrel 46 is misaligned and disengaged in this position.

Figure 5 illustrates the manual switch valve 60 released (natural position) which allows the spring element 52 and the outlet port 50 to misalign from the needle 54. Accordingly, the main housing 40 is not in fluid communication with the insulin delivery device 20. Instead, the flow path 45 is aligned to provide fluid communication between the vial adapter 44 and the barrel 46. As a result, the medicament is transferred from the vial 10 to the barrel 46.

To transfer the medicament to the insulin delivery device 20, the manual switch valve 60 is depressed as illustrated in Figure 4 to align the barrel 46, the outlet port 50 and the needle 54. The user subsequently depresses the vented plunger 72 to transfer the medicament. Accordingly, this position is used to remove air from the insulin delivery device 20 and transfer medicament to the insulin delivery device 20.

The spring element 52 is a rigid member disposed near a bottom surface of the main housing 40. As described above, the spring element 52 cooperates with the manual switch valve 60 to control the flow of the medicament. Specifically, as illustrated in Figures 2 and 3, the spring element 52 engages the spring 53 that bottoms out on a portion of the main housing 40 to ensure compression of the spring 53. This configuration allows the spring element 52 to slide into various flow path positions based on user manipulation of the manual switch valve 60.

The manual switch valve 60, as illustrated in Figures 2, 4 and 5, includes an arm 62, a pivot shaft 64, a stopping element 66 and a button 68. As described above, the manual switch valve 60 incorporates the button 68 to control the operation of the medicament filling system 30. Specifically, the button 68 is disposed on a distal end of the arm 62 adjacent to the spring element 52. Whereas, the stopping element 66 is disposed on a proximal end of the arm 62 near the top of the barrel 46.

As described below, the stopping element 66 is configured to engage and disengage one of a plurality of dial dose stops 76 to aid in dose setting. The stopping element 66 can also be disposed at the top of the barrel 46 as illustrated in Figure 2 to advantageously prevent backflow after the medicament is transferred to the insulin delivery device 20. In this manner, the user advantageously does not have to hold the vented plunger 72 down after the medicament delivery to ensure that the medicament does not leave the insulin delivery device 20.

The arm 62 rotates about a pivot shaft 64 disposed between the button 68 and the stopping element 66. The pivot shaft 64 allows the arm 62 to rotate between an engaged and disengaged position based on user activation of the button 68.

The syringe barrel components 70 includes the vented plunger 72, a dial dose 74 and the plurality of dial dose stops 76. The vented plunger 72 advantageously includes a fluid stopping membrane to prohibit the medicament from escaping the barrel 46, and a one way valve to allow air to vent from the barrel 46 while keeping the medicament in. Specifically, the vented plunger 72 allows air to exit the medicament filling system 30 when air is removed from the insulin delivery device 20. Also, when the barrel 46 is filled with medicament, the air previously disposed in the barrel 46 is released through the vented plunger 72.

The dial dose 74 is disposed on a proximal end of the vented plunger 72. The user rotates the dial dose 74 to set the desired dose. As the dial dose 74 rotates, the plunger 72 also rotates along with the plurality of dial dose stops 76 disposed on the plunger 72. Figure 3 illustrates the plurality of dial dose stops 76 rotationally positioned that each correspond to a different dosage. Preferably, the dial dose stops 76 are molded into and along an axial length of the plunger 72. Based on the set dose from the dial dose 74, one of the plurality of dial dose stops 76 aligns with the stopping element 66 to restrict the amount of medicament that enters into the barrel 46.

Some insulin delivery devices 20 may incorporate a flexible reservoir chamber such as a flexible bag, for example. These insulin delivery devices 20 may provide back pressure upon filling of medicament, as well as excess air that should be removed to provide high accuracy during medicament filling. The medicament filling system 30 disclosed herein advantageously prevents backflow of the medicament, and removes air to improving filling accuracy and control medicament delivery volume while minimizing needle exposure.

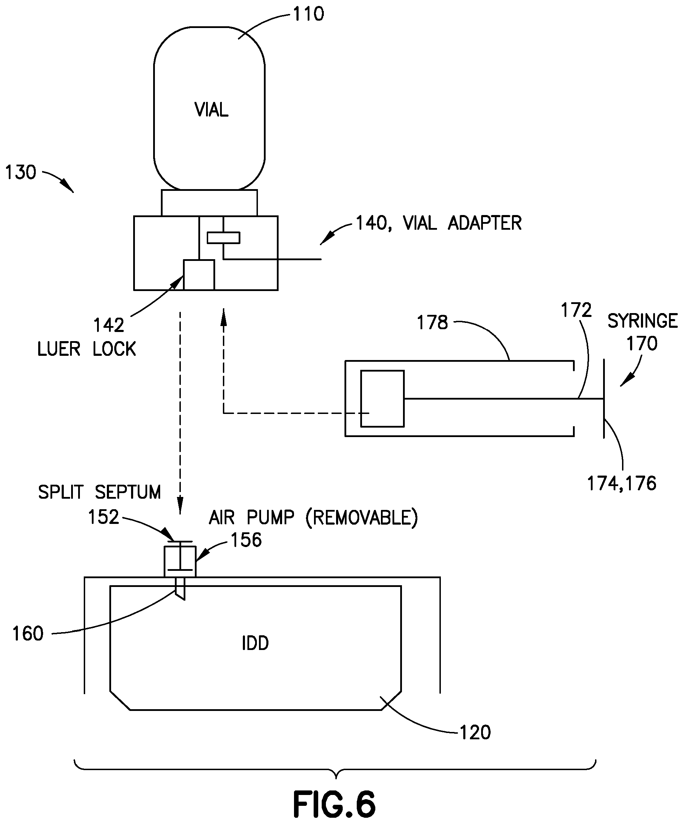

Figures 6-10 illustrate a medicament filling system 130, according to a second embodiment. Figure 6 illustrates a schematic of the medicament filling system 130 that establishes cooperation with a vial 110 and an insulin delivery device 120 as similarly described above. The medicament filling system 130 further includes a needleless vial adapter 140, an alignment platform 150, a needleless syringe 170 and a plunger lock assembly 180. The alignment platform 150 is advantageously the only component in the medicament filling system 130 that uses a needle.

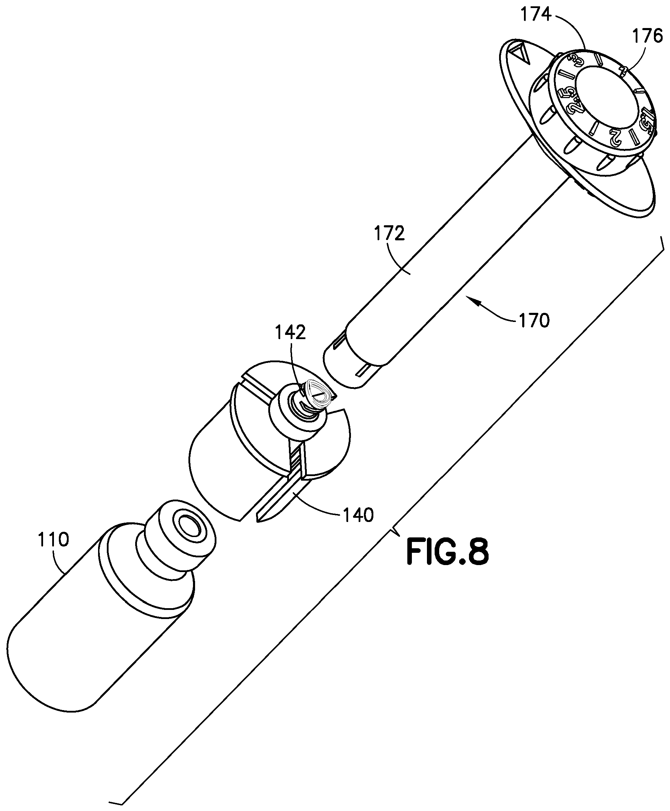

Figures 6 and 8 illustrates that a proximal end of the vial adapter 140 includes a luer lock 142. The proximal end of the vial adapter 140 is configured to engage the syringe 170 to establish fluid communication. The distal end of the vial adapter 140 is configured to pierce a septum in the vial 110 via a needle (not shown) to establish fluid communication.

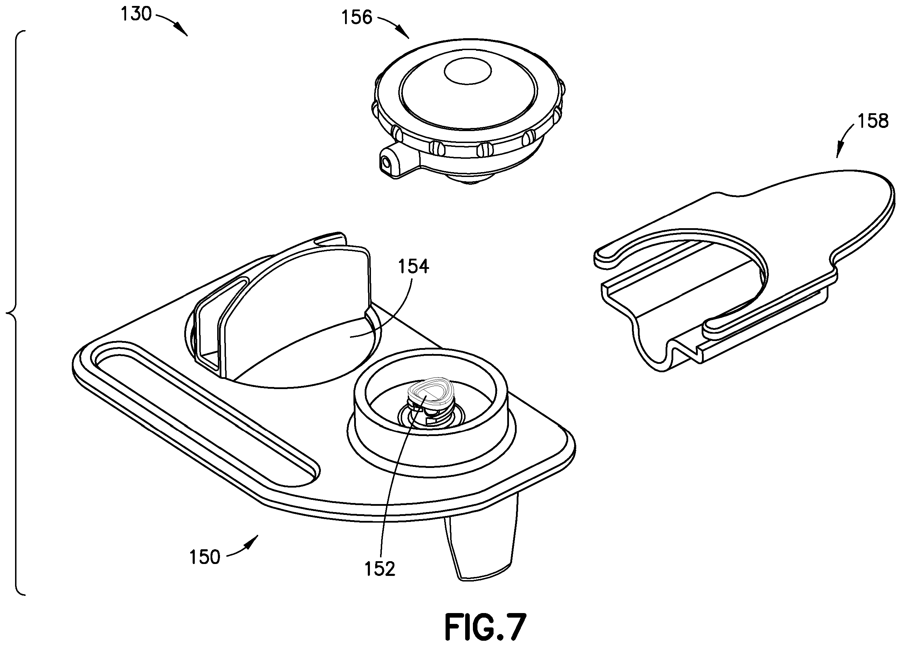

Figures 6 and 7 illustrate the alignment platform 150. An underside surface of the alignment platform 150 advantageously provides alignment for mounting purposes and is configured to engage the insulin delivery device 120. The alignment platform 150 includes a split septum 152, a fixture cavity 154, an air pump 156, a needle cover 158 and a needle 160. The split septum 152 provides selective fluid communication with the air pump 156 and the syringe 170. The fixture cavity 154 provides a space to use a fixture for mounting the alignment platform 150 and providing stability.

The air pump 156 is different from the one used in the embodiment described above. Specifically, the air pump 156 is preferably automatically operated but can also be manually operated. The air pump 156 includes two one-way valves and a collapsible chamber such as a bulb, bellow or piston-barrel. The air pump 156 is configured to engage the split septum 152 and remove air from the insulin delivery device 120 prior to filling with medicament. The needle 160, as illustrated in Figure 6, is disposed on an underside surface of the alignment platform 150 and in-line with the split septum 152. The needle 160 is configured to pierce a septum of the insulin delivery device 120 to establish fluid communication with the alignment platform 150.

The needle cover 158 is used to cover the needle 160 and engage the alignment platform 150. As illustrated in Figure 7, the needle cover 158 is uniquely shaped to surround the split septum 152 and luer connection while engaging both a top surface and the underside surface of the alignment platform 150 to ultimately cover and enclose the needle 160.

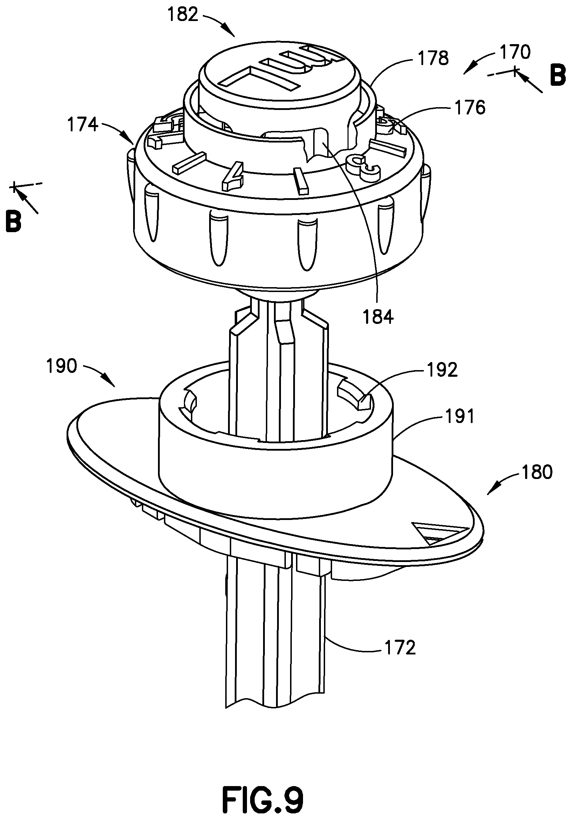

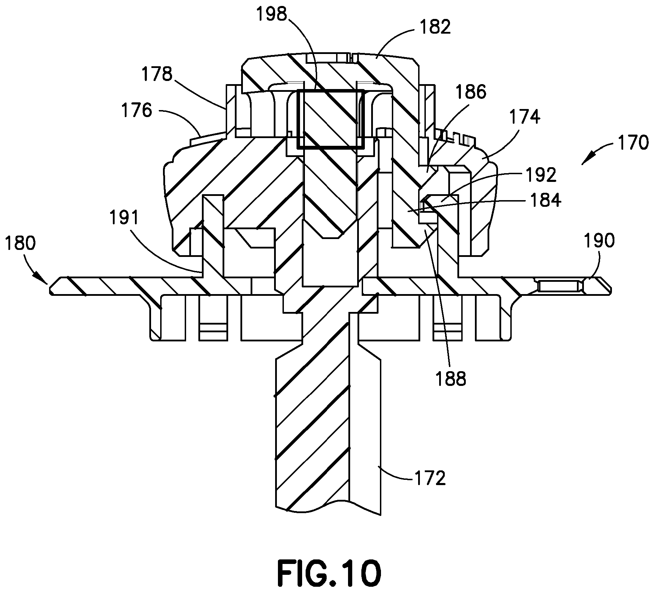

Figure 8 further illustrates the syringe 170 having a plunger 172, a plunger head 174, a dial dose 176 and a syringe barrel (not shown) as conventionally understood by one skilled in the art. Figures 9 and 10 illustrate the plunger lock assembly 180 that cooperates with the syringe 170. The plunger lock assembly 180 advantageously locks the plunger 172 when all the medicament is dispensed from the syringe barrel to prevent backflow of the medicament back into the syringe barrel.

The plunger lock assembly 180 includes a lock button 182, a locking flange 184 having first and second protrusions 186, 188, a lock base 190, a circular extrusion 191, a base locking flange 192 and a compression spring 198. The lock button 182 is advantageously and conveniently disposed above the plunger head 174 of the plunger 172. The lock button 182 is button shaped and includes a plurality of legs or locking flanges 184 that extend through cavities in the plunger head 174.

As illustrated in Figures 9 and 10, the syringe 170 further includes an anti-accidental push ring 178. The anti-accidental push ring 178 includes an elevated surface surrounding the lock button 182 to advantageously prevent inadvertent depression of the lock button 182. In one embodiment, the anti-accidental push ring 178 includes a continuous wall surrounding the lock button 182. In another embodiment, the anti-accidental push ring 178 includes a wall having gaps spaced out around the lock button 182.

If the user drops the syringe 170, for example, the lock button 182 will not be depressed beyond a top surface of the anti-accidental push ring 178 to safely prevent incidental triggering. The anti-accidental push ring 178 is designed to require a deliberate depression of the lock button 182 that extends into the anti-accidental push ring 178. Such a configuration can advantageously avoid the accidental triggering of the lock button 182 of the plunger lock assembly 180 while allowing a finger of the user to operate the syringe 170.

The plurality of locking flanges 184 each include the first protrusion 186 and the second protrusion 188. The first protrusion 186 secures the plunger lock assembly 180 to the plunger head 174 of the syringe 170. The second protrusion 188 secures the lock button 182 to a lock base 190 when in a locked position.

As illustrated in Figure 9, the lock base 190 is fixed to syringe barrel, preferably by a snap locking arrangement, although other fixing means are contemplated herein. The lock base 190 includes a circular extrusion 191 extending upward and toward the plunger head 174. A plurality of base locking flanges 192 is disposed in an inner diameter and at a proximal end of the circular extrusion 191. The plurality of base locking flanges 192 engage the second protrusions 188 of the lock button 182 to lock the plunger 172 after the medication is dispensed. The plunger head 174 can also be rotated from a locked position to disengage the plunger 172 from the lock button 182 and enter into the unlocked position.

A compression spring 198 is disposed between the lock button 182 and the plunger head 174 to keep the lock button 182 in the unlocked position before the user compresses the lock button 182 into the locked position. Thus, in the unlocked position, the compression spring 198 is compressed to allow the plunger 172 to operate. However, in the locked position after the medication is dispensed, the compression spring 198 is further compressed to cause the plurality of base locking flanges 192 to engage with the second protrusions 188 of the lock button 182.

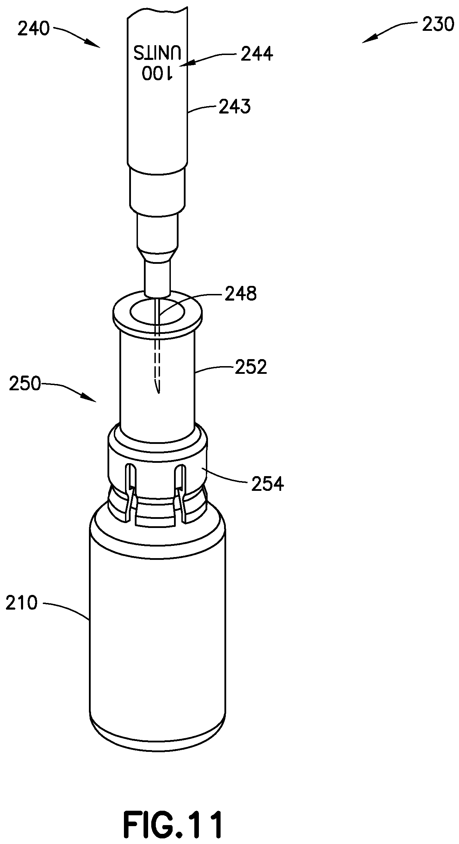

Figures 11-15 illustrate a syringe assembly 230, according to a third embodiment, that fills a syringe 240 with medicament and dispenses the medicament into an insulin delivery device 220. The syringe assembly 230 includes the syringe 240 having well-known components such as a flange 242 that allows a user to grasp the syringe 240, a barrel 243 that carries medicament, a dose indicator 244 that provides a visual indication of the amount of the medicament in the syringe 240, a plunger 245 that moves the medicament into and out of the barrel 243, a plunger head 246 that provides a space for finger depression/actuation and a needle 248 upon which the medicament flows.

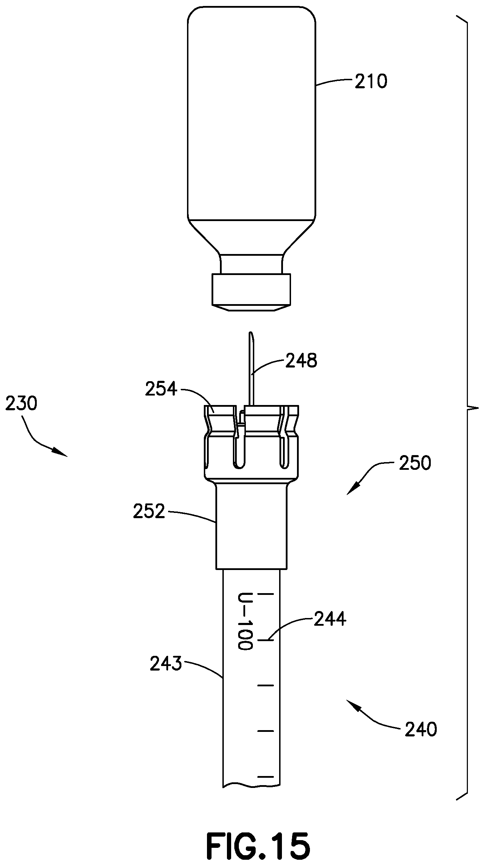

The syringe assembly 230 further includes an adapter 250 that provides alignment between the syringe 240 and a vial 210, as well as alignment between the syringe 240 and an insulin delivery device 220. The adapter 250 also allows the user to hold the vial 210 and the syringe 240 together when engaged. Figure 11 illustrates the syringe 230 in the process of engaging the adapter 250, and Figure 15 illustrates the syringe 230 disengaged from the adapter 250. The adapter 250 includes an alignment tube 252 that aids in aligning the syringe 240 to the vial 210 or to the insulin delivery device 220.

The alignment tube 252 is a larger target compared to the syringe 240. As a result, the alignment tube 252 advantageously allows the user to more efficiently aim the syringe 240 and the adapter 250 into engagement with the vial 210 or into engagement with the insulin delivery device 220. The improved aiming efficiency also advantageously reduces accidental bending of the needle. A locking mechanism 254, preferably a snap lock or threads, is disposed on a distal end of the adapter 250 to engage the vial 210 and the insulin delivery device 220.

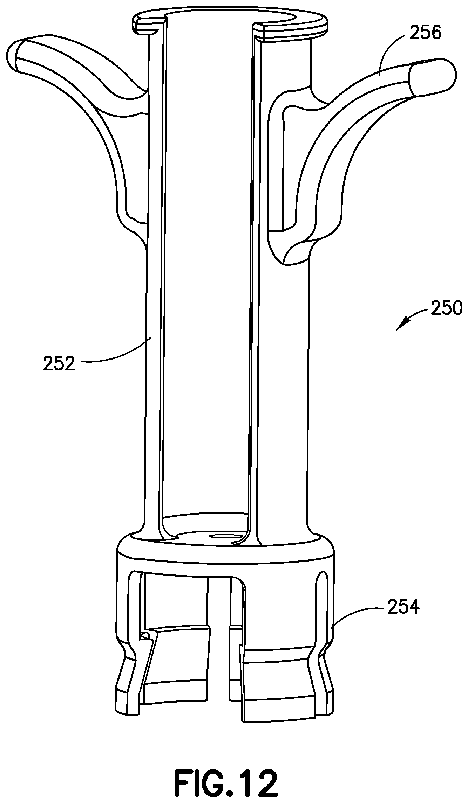

Figures 12-14 illustrate several embodiment of the adapter 250 providing various advantageous features related to ergonomics and controlling needle depth. Figure 12 illustrates two arms 256 on the alignment tube 252 of the adapter 250 that protrude outwardly from a centerline of the alignment tube 252 to advantageously aid in ergonomically handling and controlling the syringe assembly 230.

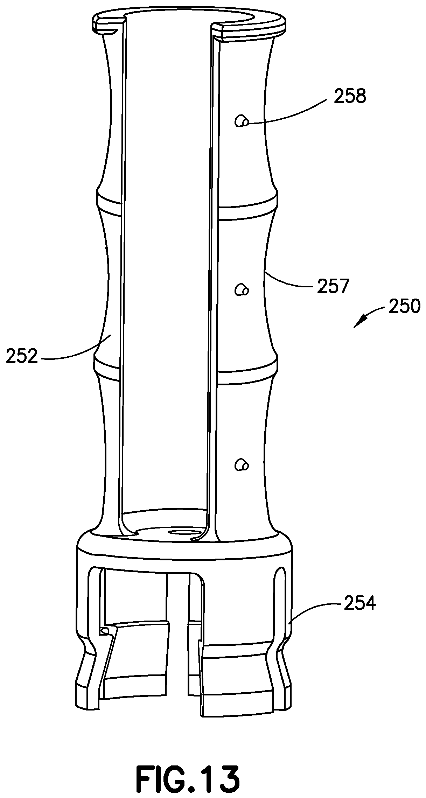

Figure 13 illustrates a plurality of curved depressions 257, each having a protrusion 258 centrally located. The curved depression 257 is advantageously configured to ergonomically engage a finger of the user and the protrusion 258 advantageously provides friction for the user to maintain grip of the adapter 250.

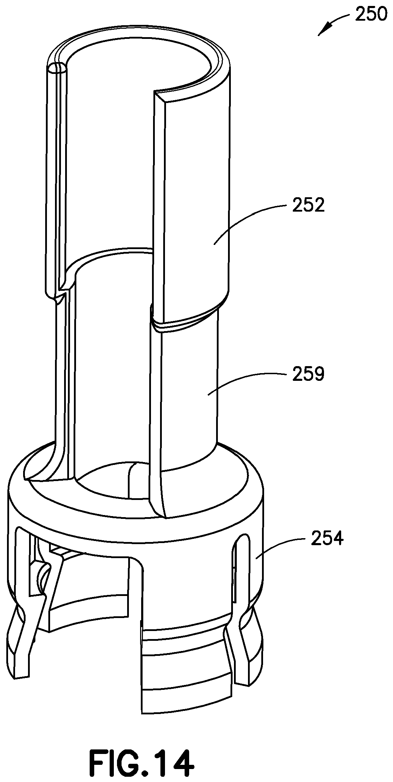

Figure 14 illustrates a needle depth control mechanism 259. The alignment tube 252 is replaced by, or preferably cooperates with the needle depth control mechanism 259. The needle depth control mechanism 259 includes a sleeve assembly having two or more sleeves that are collinear and rotate with respect to each other. The sleeve assembly functions as a telescoping device that reduces an inner diameter when rotated, similarly to a telescoping pole.

The needle depth control mechanism 259 advantageously controls needle penetration into the vial 210 and the insulin delivery device 220, and reduces needle stick. This configuration is especially advantageous to maximize extraction of the medicament from the vial 210 since a shorter needle can recover more of the medicament. Also, the sleeve assembly of the needle depth control mechanism 259 is collapsible to store the adapter 250 in compact and safe spaces.

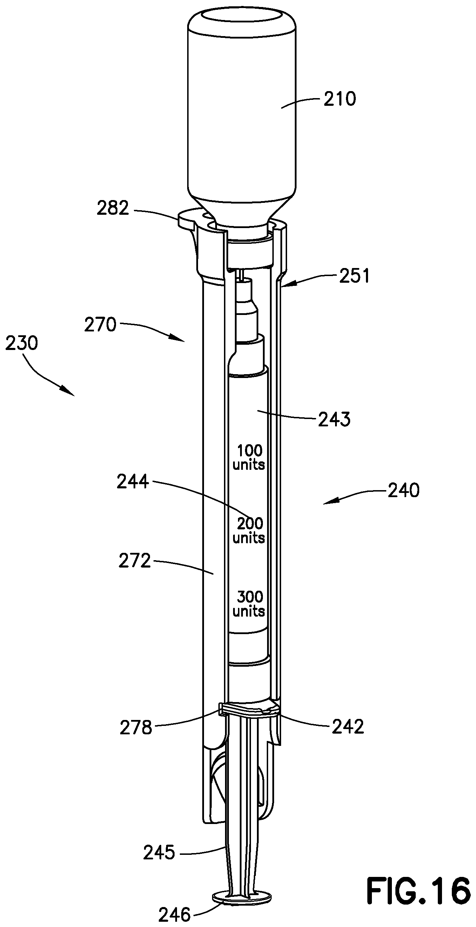

Figures 16-18 illustrate a syringe assembly 230, according to a fourth embodiment, as similarly described above but includes an alternate adapter 251. The adapter 251 of this embodiment is integrated into a plunger lock assembly 270. The plunger lock assembly 270 includes a housing 272, a flange slot 278, a plunger lock 280 and a mounting flange 282.

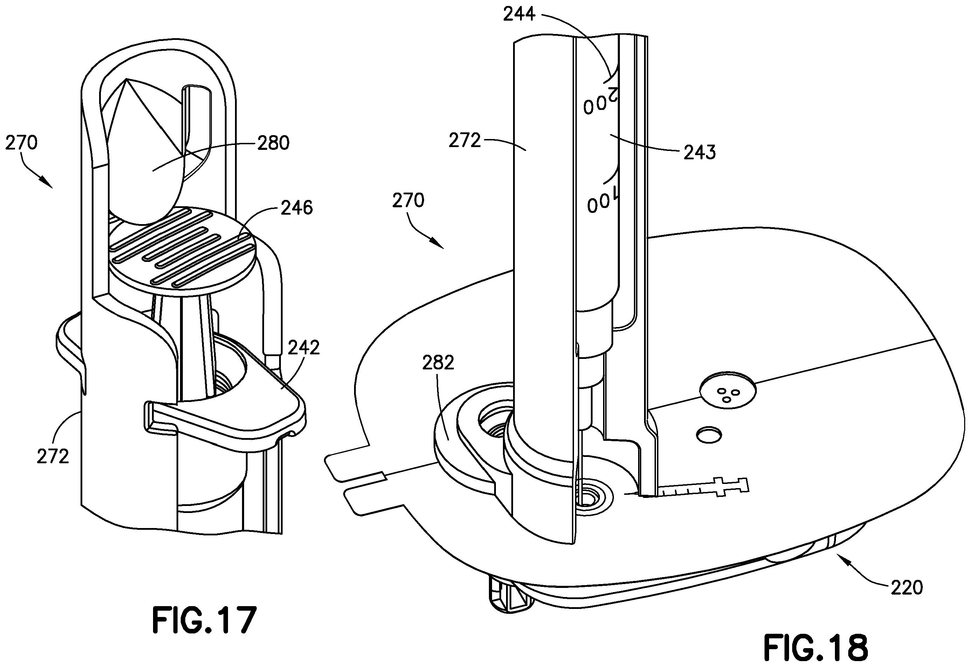

The housing 272 of the plunger lock assembly 270 carries or encloses the syringe assembly 230. The flange slot 278 is a cavity that engages the flange 242 of the syringe 240. Figure 17 illustrates that the plunger lock 280 is disposed above the plunger head 246 after the medication is dispensed to prevent backflow from reentering the syringe 240. Figures 16 and 18 illustrate the mounting flange 282 disposed at a distal end of the plunger lock assembly 270 to provide engagement to the vial 210 and also provide a mounting surface for alignment when engaging the insulin delivery device 220.

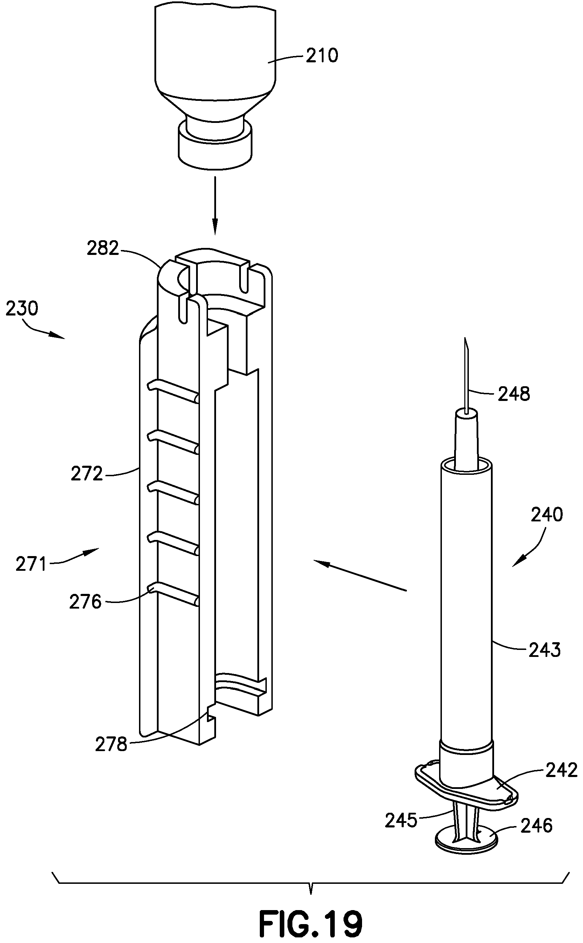

Figures 19-21 illustrate a syringe assembly 230, according to a fifth embodiment without an adapter and with a second embodiment of a plunger lock assembly 271. The plunger lock assembly 271 includes some of the features described above and further includes a dose window 274 and a grip surface 276. Specifically, the housing 272 of the plunger lock assembly 271 includes a grip surface 276 for the user to securely grasp the plunger lock assembly 271.

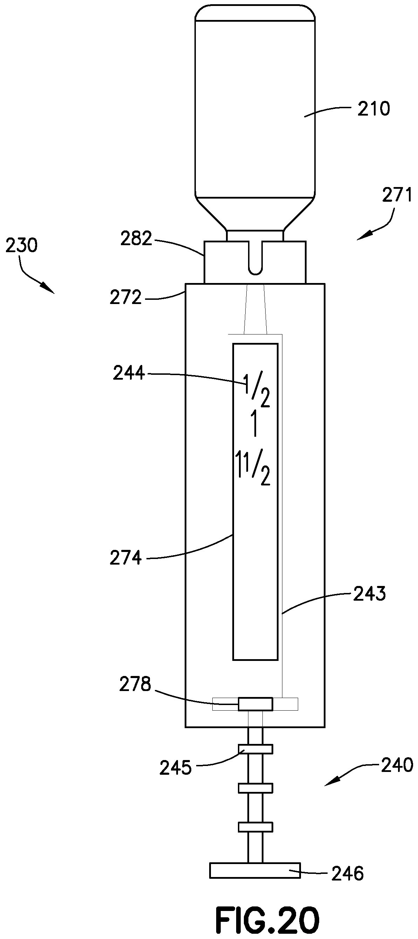

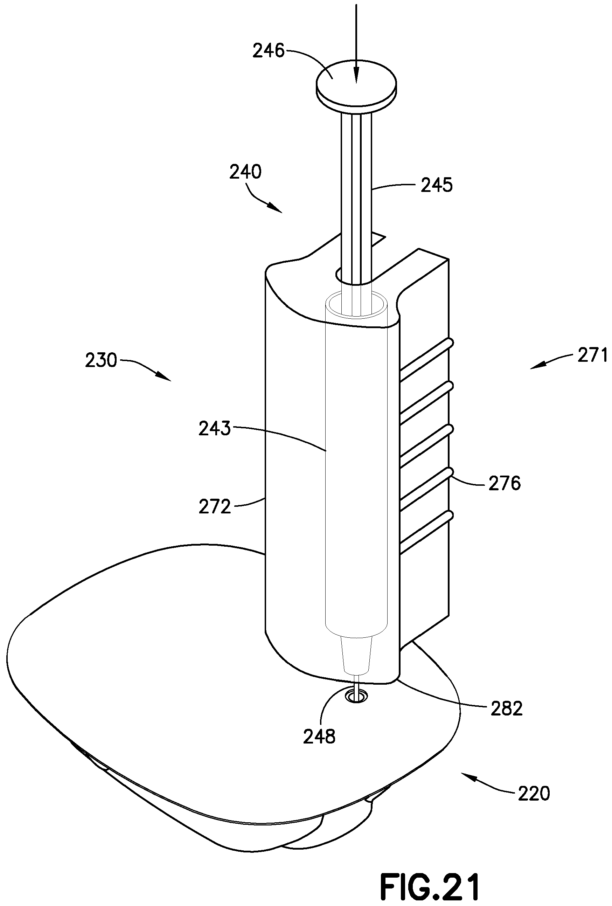

Figure 20 illustrates the syringe 240 in the plunger lock assembly 271 engaged to the vial 210. The dose window 274 on the housing 272 of the plunger lock assembly 271 is advantageously magnified to allow the user to conveniently see the dosage amount in the barrel 243 of the syringe 240 and the gradual movement of the plunger 245 during filling and dispensing. Figure 21 illustrates the syringe 240 in the plunger lock assembly 271 engaged to the insulin delivery device 220 to transfer the medicament.

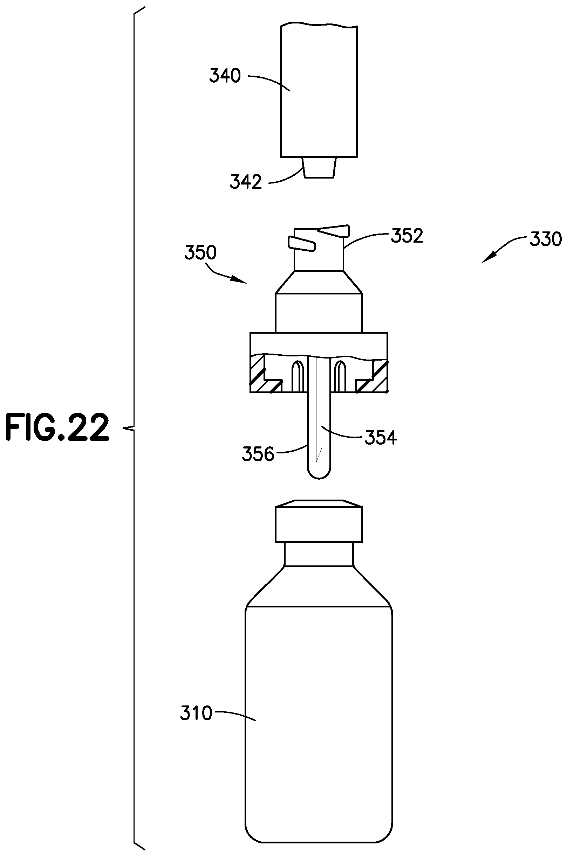

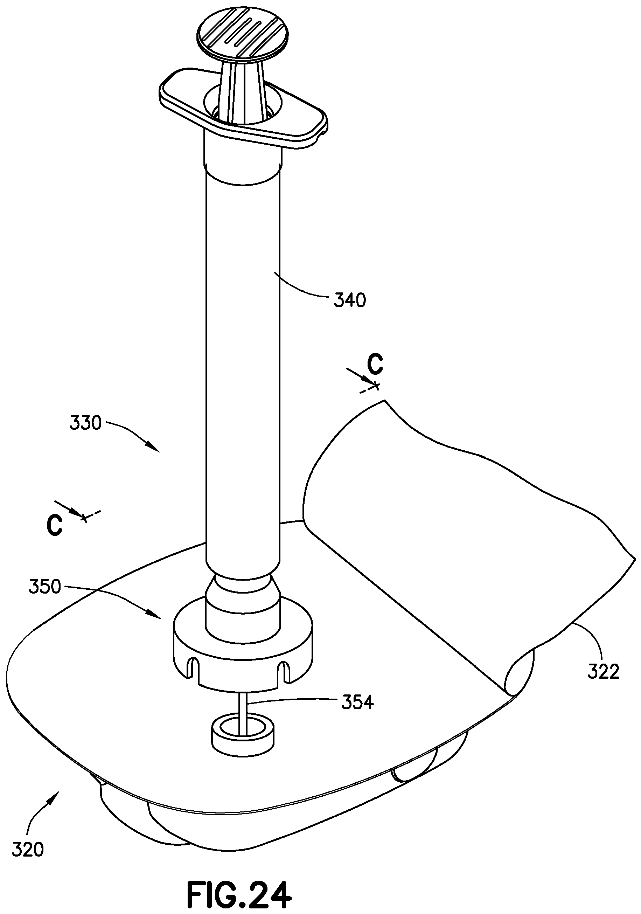

Figures 22-25 illustrates a syringe assembly 330, according to a sixth embodiment. In this embodiment, a vial 310 and an insulin delivery device 320 are similarly described above. Figure 24 illustrates the insulin delivery device 320 including a septum 324 and a film cover 322 to enclose the insulin delivery device 320 and the septum 324 prior to use. The septum 324 provides selective fluid communication to the insulin delivery device 320.

The syringe assembly 330 includes a needleless syringe 340 and a syringe connector 342. It is advantageous to use a needless syringe 340 to reduce needle sticking and improve safety. The syringe connector 342 is preferably a luer connector but other forms of engagement are contemplated.

The syringe assembly 330 further includes an adapter 350 configured to engage the vial 310 and the insulin delivery device 320. The adapter 350 includes an adapter connector 352 that is configured to engage the syringe connector 342. A needle 354 is disposed on a distal end of the adapter 350 to engage a septum of the vial 310 and the insulin delivery device 320. Prior to use, the needle 354 is enclosed by a rubber sleeve 356 to protect the needle 354 from inadvertent use.

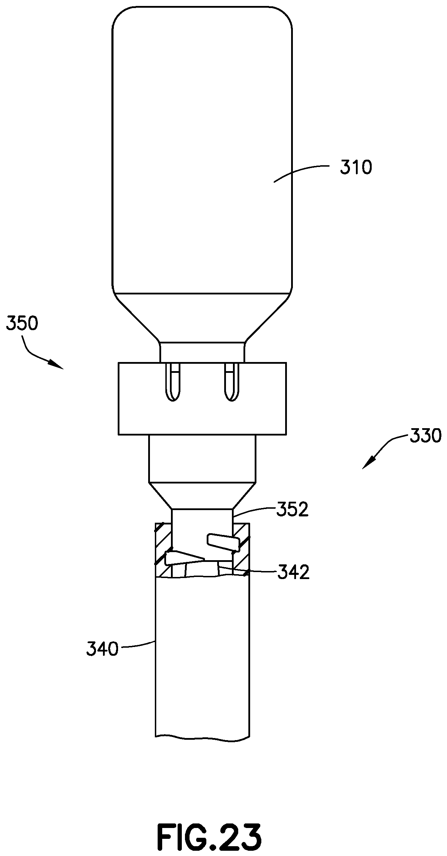

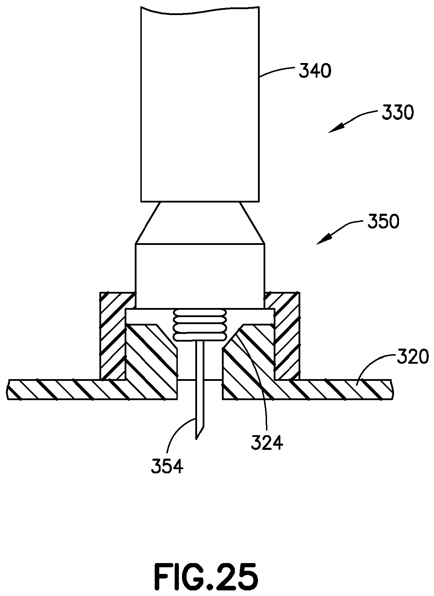

Figure 23 illustrates the syringe 330 connected to the adapter 350 and engaged to the vial 310 to fill the syringe 330 with medicament. After filling, Figure 25 illustrates the syringe 330 connected to the adapter 350 and engaged to the insulin delivery device 320. Specifically, the needle 354 of the adapter 350 pierces the septum 324 of the insulin delivery device 320 to establish fluid communication.

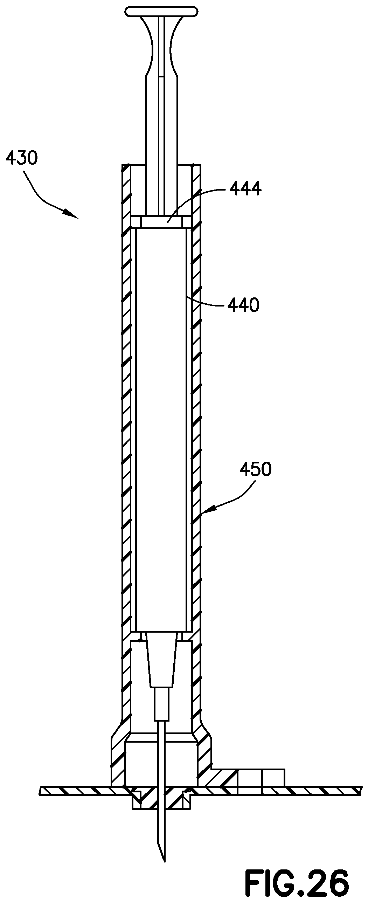

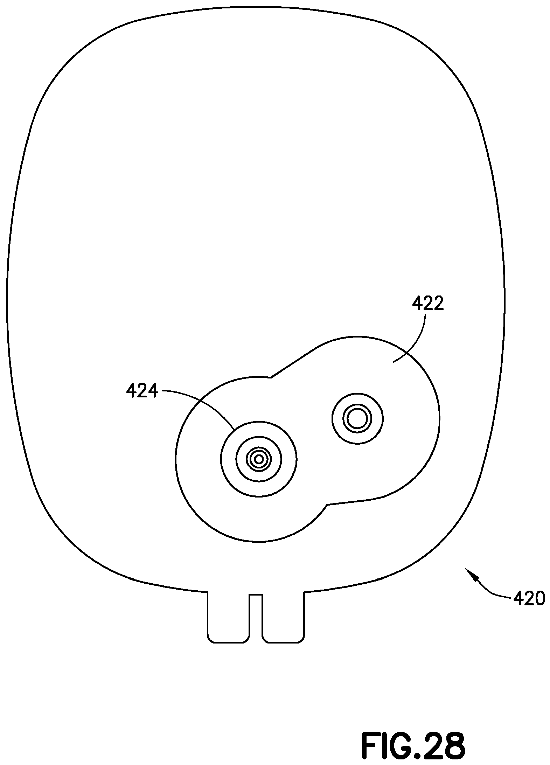

Figures 26-28 illustrates a syringe assembly 430, according to a seventh embodiment. In this embodiment, a vial 410 and an insulin delivery device 420 are similarly described above. However, Figure 28 illustrates that the insulin delivery device 420 further includes an alignment footprint 422 and a needle port 424. The alignment footprint 422 comprises a profile that is printed on a label of the insulin delivery device 420 to guide engagement of the syringe assembly 430. The needle port 424 is a hole alignment feature to provide mechanical alignment between the insulin delivery device 420 and the syringe assembly 430.

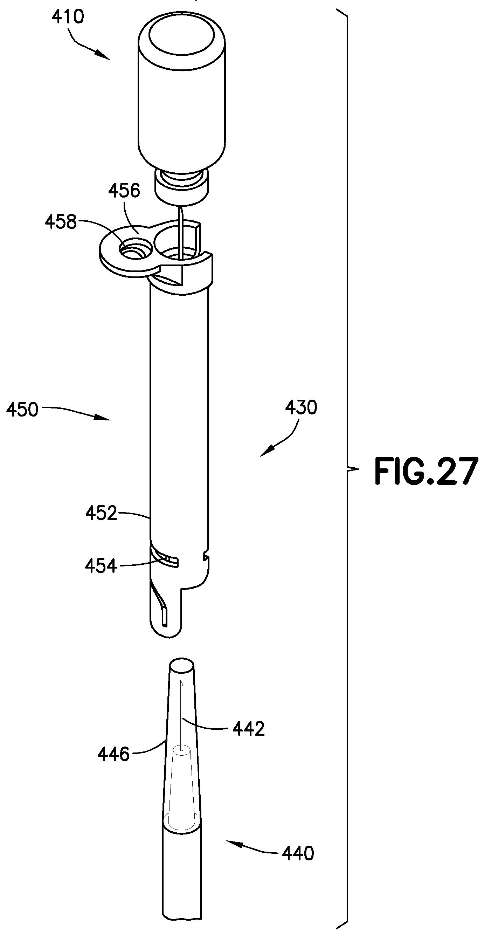

The syringe assembly 430 includes a syringe 440 having a needle 442, a flange 444 and a needle cover 446 as conventionally understood by one skilled in the art. Figure 26 illustrates the syringe 440 being disposed in an adapter 450. Figure 27 illustrates an exploded view of the syringe assembly 430 where the adapter 450 includes a housing 452, a slot 454, a base 456 and a hole 458.

The housing 452 is configured to carry the syringe 440. The slot 454 is configured to engage the flange 444 of the syringe 440 to secure the engagement. The base 456 is used to mount onto the insulin delivery device 420 and is also configured to engage the vial 410. As illustrated in Figures 27 and 28, the base 456 is positioned on the alignment footprint 422 of the insulin delivery device 420 so that proper alignment and engagement can take place. The hole 458 in the base 456 is used to provide mechanical alignment with the needle port 424 of the insulin delivery device 420.

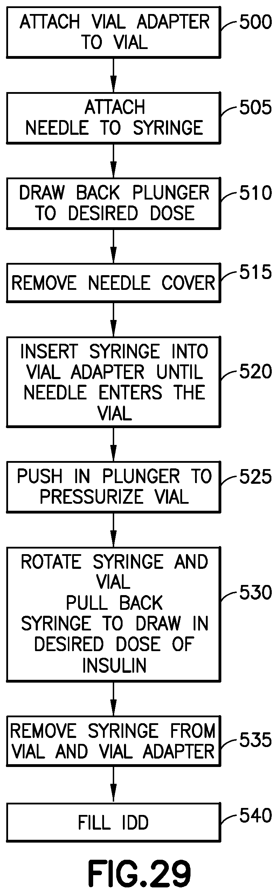

Figure 29 illustrates a flow diagram for a user to transfer medicament from a vial into the syringe assembly in the plurality of relevant embodiments described above using an adapter. This transfer takes place prior to expelling the medicament into the delivery device. Step 500 attaches a vial adapter to a vial. Step 505 attaches a needle to a syringe. Step 510 retracts a plunger to a desired dose. Step 515 moves a needle cover. Step 520 inserts a syringe into a vial adapter until the needle enter the vial. Step 525 depresses the plunger to provide air pressure into the vial. Step 530 rotates the syringe and vial such that the vial is above the syringe. Subsequently, the user retracts the plunger to draw in a desired dose of medicament. Step 535 removes the syringe from the vial and the vial adapter. In step 540, the syringe is now ready to fill the insulin delivery device.

The foregoing detailed description of the certain exemplary embodiments has been provided for explaining the principles of the invention and its practical application, thereby enabling others skilled in the art to understand the invention for various embodiments and with various modifications as are suited to the particular use contemplated. This description is not necessarily intended to be exhaustive or to limit the invention to the precise embodiments disclosed. In addition, any of the embodiments, features and/or elements disclosed herein may be combined with one another to form various additional combinations not specifically disclosed, as long as the embodiments, features and/or elements being combined do not contradict each other. Accordingly, additional embodiments are possible and are intended to be encompassed within this specification and the scope of the invention, as long as they fall under the appended claims.
The specification describes specific examples to accomplish a more general goal that may be accomplished in another way.

As used in this application, the terms "front," "rear," "upper," "lower," "upwardly," "downwardly," and other orientational descriptors are intended to facilitate the description of the exemplary embodiments of the present invention, and are not intended to limit the structure of the exemplary embodiments of the present invention to any particular position or orientation. Terms of degree, such as "substantially" or "approximately" are understood by those of ordinary skill to refer to reasonable ranges around and including the given value, for example, general tolerances associated with manufacturing, assembly, and use of the described embodiments.

## Claims

1. A medicament filling system (30) that provides medicament to a delivery device (20), the medicament filling system comprising:
a main housing (40) having:
an air pump (42);
a vial adapter (44) that is configured to engage a vial (10) containing medicament, the vial adapter being in communication with the air pump;
a barrel (46) being in selective communication with the vial adapter and the delivery device; and
a valve (60) that locks and unlocks movement of a plunger (72) in the barrel, **characterized in that**
the valve includes a button connected to a stopping element that engages and disengages the plunger in the barrel; wherein
when the button is depressed, the plunger in the barrel is disengaged from the stopping element,
wherein the valve further includes:
an arm including the stopping element (66) on a distal end of the arm; and
a pivot shaft;
wherein the arm rotates with respect to the pivot shaft to engage and disengage the stopping element to the plunger in the barrel.

2. The medicament filling system of claim 1, wherein:
the valve (60) is unlocked for the barrel to remove air from the delivery device;
the valve is locked to prevent the barrel from receiving the medicament from the vial adapter beyond a preset dose;
the valve is unlocked to deliver the medicament from the barrel to the delivery device; and
the valve is locked after the medicament delivery to prevent backflow from the delivery device into the barrel.

3. The medicament filling system of claim 1, wherein:
the plunger (72) of the barrel (70) vents air from the barrel;
the plunger includes a dial dose (74) at a proximal end of the plunger and corresponding dial dose stop (76) positions to indicate dosage; and
the barrel includes a viewing window (48) to indicate an amount of the medicament disposed in the barrel.

4. A method of using a medicament filling system (30) in accordance with any of claims 1-3 to fill medicament into a
delivery device (20), the method comprising:
attaching the medicament filling system to the delivery device;
establishing fluid communication between the delivery device and the medicament filling system;
connecting a vial (10) to the medicament filling system to establish fluid communication;
setting a dose on a plunger (72) of a barrel (70) in the medicament filling system;
releasing the barrel via a valve to allow for operation of the plunger;
pulling the plunger to remove air from the delivery device;
locking the plunger via the valve to prevent movement of the plunger and the barrel;
transferring the medicament from the vial to the barrel;
depressing the valve to release the plunger for operation; and
filling the delivery device with medicament.

5. The method of claim 4, comprising rotating the plunger of the barrel to set the dosage.

6. The method of claim 4, comprising piercing a septum in the delivery device to establish fluid communication to the medicament filling system.

7. The method of claim 4, comprising pumping air into the vial to transfer the medicament from the vial to the barrel.

8. The method of claim 4, comprising venting excess air from the plunger of the barrel.

## Patentansprüche

1. Medikamentenabfüllsystem (30) zum Abfüllen einer Abgabevorrichtung (20) mit Medikamenten, wobei das Medikamentenabfüllsystem umfasst:
ein Hauptgehäuse (40) mit:
einer Luftpumpe (42);
einem Phiolenadapter (44), der dazu ausgebildet ist, mit einer Phiole (10), die ein Medikament enthält, in Eingriff zu treten, wobei der Phiolenadapter mit der Luftpumpe in Verbindung steht;
einem Zylinder (46), der in selektiver Verbindung mit dem Phiolenadapter und der Abgabevorrichtung steht; und
einem Ventil (60), das eine Bewegung eines Kolbens (72) in dem Zylinder verriegelt und entriegelt,
**dadurch gekennzeichnet, dass** das Ventil einen Knopf aufweist, der mit einem Stoppelement verbunden ist, das mit dem Kolben in Eingriff bringbar und aus dem Eingriff lösbar ist; wobei
wenn der Knopf gedrückt wird, der Kolben im Zylinder außer Eingriff mit dem Stoppelement gebracht wird,
wobei das Ventil ferner umfasst:
einen Arm, der an seinem distalen Ende das Stoppelement (66) trägt; und
eine Drehachse;
wobei der Arm relativ zur Drehachse drehbar ist, um das Stoppelement mit dem Kolben im Zylinder in Eingriff zu bringen und aus dem Eingriff zu lösen.

2. Medikamentenabfüllsystem nach Anspruch 1, wobei:
das Ventil (60) entriegelt wird, damit der Zylinder Luft aus der Abgabevorrichtung entfernen kann;
das Ventil verriegelt wird, damit der Zylinder kein Medikament aus dem Phiolenadapter über eine voreingestellte Dosis hinaus aufnehmen kann;
das Ventil entriegelt wird, damit das Medikament aus dem Zylinder in die Abgabevorrichtung abgegeben werden kann; und
das Ventil nach Abgabe des Medikaments verriegelt wird, damit kein Rückfluss aus der Abgabevorrichtung in den Zylinder erfolgen kann.

3. Medikamentenabfüllsystem nach Anspruch 1, wobei:
der Kolben (72) des Zylinders (70) Luft aus dem Zylinder entlüftet;
der Kolben eine Einstelldosis (74) an einem proximalen Ende des Kolbens und entsprechende, in unterschiedlichen Positionen angeordnete Einstelldosisanschläge (76) aufweist, um die Dosis anzuzeigen; und
der Zylinder ein Sichtfenster (48) zur Anzeige einer im Zylinder befindlichen Medikamentmenge umfasst.

4. Verfahren zum Verwenden eines Medikamentenabfüllsystems (30) nach einem der Ansprüche 1 bis 3 zum Abfüllen von Medikamenten in eine Abgabevorrichtung (20), wobei das Verfahren umfasst:
Anbringen des Medikamentenabfüllsystems an der Abgabevorrichtung;
Herstellen einer Fluidverbindung zwischen der Abgabevorrichtung und dem Medikamentenabfüllsystem;
Anschließen einer Phiole (10) an das Medikamentenabfüllsystem, um eine Fluidverbindung herzustellen;
Einstellen einer Dosis an einem Kolben (72) eines Zylinders (70) in dem Medikamentenabfüllsystem;
Freigeben des Zylinders über ein Ventil, um eine Betätigung des Kolbens zu ermöglichen;
Zurückziehen des Kolbens, um Luft aus der Abgabevorrichtung zu entfernen;
Verriegeln des Kolbens über das Ventil, um eine Bewegung des Kolbens und des Zylinders zu verhindern;
Überführen des Medikaments aus der Phiole in den Zylinder;
Drücken des Ventils, um den Kolben zur Betätigung freizugeben; und
Abfüllen der Abgabevorrichtung mit Medikamenten.

5. Verfahren nach Anspruch 4, umfassend Drehen des Kolbens des Zylinders, um die Dosis einzustellen.

6. Verfahren nach Anspruch 4, umfassend Durchstechen eines Septums in der Abgabevorrichtung, um eine Fluidverbindung mit dem Medikamentenabfüllsystem herzustellen.

7. Verfahren nach Anspruch 4, umfassend Einpumpen von Luft in die Phiole, um das Medikament aus der Phiole in den Zylinder zu überführen.

8. Verfahren nach Anspruch 4, umfassend Entlüften von überschüssiger Luft über den Kolben des Zylinders.

## Revendications

1. Système de remplissage de médicament (30) qui fournit un médicament à un dispositif d'administration (20), le système de remplissage de médicament comprenant :
un boîtier principal (40) comportant :
une pompe à air (42) ;
un adaptateur de flacon (44) qui est configuré pour coopérer avec un flacon (10) contenant un médicament, l'adaptateur de flacon étant en communication avec la pompe à air ;
un corps (46) qui est en communication sélective avec l'adaptateur de flacon et avec le dispositif d'administration ; et
une vanne (60) qui verrouille et déverrouille un mouvement d'un piston (72) dans le corps,
**caractérisé en ce que** la vanne comprend un bouton relié à un élément d'arrêt qui s'accouple au piston situé dans le corps et s'en désaccouple ; dans lequel
lorsque le bouton est enfoncé, le piston situé dans le corps est désaccouplé de l'élément d'arrêt,
dans lequel la vanne comprend en outre :
un bras comprenant l'élément d'arrêt (66) sur une extrémité distale du bras ; et
un axe de pivot ;
dans lequel le bras tourne par rapport à l'axe de pivot pour accoupler l'élément d'arrêt au piston situé dans le corps et le désaccoupler.

2. Système de remplissage de médicament selon la revendication 1, dans lequel :
la vanne (60) est déverrouillée pour permettre au corps d'évacuer de l'air du dispositif d'administration ;
la vanne est verrouillée pour empêcher que le corps ne reçoive le médicament en provenance de l'adaptateur de flacon en une quantité supérieure à une dose prédéfinie ;
la vanne est déverrouillée pour délivrer le médicament du corps au dispositif d'administration ; et
la vanne est verrouillée après la délivrance du médicament pour empêcher un reflux du dispositif d'administration dans le corps.

3. Système de remplissage de médicament selon la revendication 1, dans lequel :
le piston (72) du corps (70) évacue l'air du corps ;
le piston comprend un doseur à cadran (74) à une extrémité proximale du piston et correspondant à des positions d'arrêt de doseur à cadran (76) pour indiquer un dosage ; et
le corps comprend une fenêtre de visualisation (48) pour indiquer une quantité du médicament disposé dans le corps.

4. Procédé d'utilisation d'un système de remplissage de médicament (30) selon l'une quelconque des revendications 1 à 3 pour remplir un dispositif d'administration (20) d'un médicament, le procédé comprenant les étapes consistant à :
fixer le système de remplissage de médicament au dispositif d'administration ;
établir une communication fluidique entre le dispositif d'administration et le système de remplissage de médicament ;
relier un flacon (10) au système de remplissage de médicament pour établir une communication fluidique ;
régler une dose sur un piston (72) d'un corps (70) dans le système de remplissage de médicament ;
libérer le corps par le biais d'une vanne pour permettre une mise en œuvre du piston ;
tirer le piston pour évacuer de l'air du dispositif d'administration ;
verrouiller le piston par le biais de la vanne pour empêcher un mouvement du piston et du corps ;
transférer le médicament du flacon au corps ;
enfoncer la vanne pour libérer le piston à des fins de mise en œuvre ; et
remplir le dispositif d'administration du médicament.

5. Procédé selon la revendication 4, comprenant en outre l'étape consistant à tourner le piston du corps pour régler le dosage.

6. Procédé selon la revendication 4, comprenant en outre l'étape consistant à percer un septum dans le dispositif d'administration pour établir une communication fluidique avec le système de remplissage de médicament.

7. Procédé selon la revendication 4, comprenant en outre l'étape consistant à pomper de l'air dans le flacon pour transférer le médicament du flacon au corps.

8. Procédé selon la revendication 4, comprenant en outre l'étape consistant à évacuer de l'air en excès à partir du piston du corps.
